# NEW EUROPEAN PATENT SPECIFICATION

(11) **EP 0 719 122 B2**
(45) Date of publication and mention of the opposition decision: **23.08.2006**
(45) Mention of the grant of the patent: 23.05.2001
(21) Application number: 94927143.1
(22) Date of filing: 08.09.1994
(51) Int. Cl.: A61F 13/15

(54) **ABSORPTION BODY**
ABSORPTIONSELEMENT
CORPS D'ABSORPTION

(30) Priority: 13.09.1993 SE 9302958
(43) Date of publication of application: 03.07.1996
(73) Proprietor: SCA Hygiene Products AB, 405 03 Göteborg (SE)
(72) Inventor: LINDQUIST, Bengt, S-443 51 Lerum (SE); VASTAG, Eva, S-438 08 Härryda (SE); JOHANSSON, Dan, S-426 52 Västra Frölunda (SE)
(74) Representative: Romare, Laila Anette
(86) International application number: PCT/SE1994/000835
(87) International publication number: WO 1995/007673

(56) References cited:
- EP-A- 0 193 309
- EP-A- 0 523 683
- CH-A- 414 499
- DE-B- 1 163 491
- SE-B- 433 430
- US-A- 3 881 490

## Description

### TECHNICAL FIELD:

The present invention relates to an absorption body, suitable for use in sanitary napkins or similar, according to claim 1, and a method of manufacturing an absorption body according to the preamble of claim 8.

### BACKGROUND OF THE INVENTION:

In order to fit comfortably to the wearer, sanitary napkins are often formed with an hour-glass shaped absorbent pad having a narrow waist region which fits between tne thighs of the wearer. Due to the narrowness of the pad at the region at which discharged body fluid is first intended to contact the pad, there is an increased risk of leakage of body fluid at the side edges of the napkin in use. Many solutions have been proposed to address this problem.

EP-A-0 249 405 describes a sanitary napkin having a raised thickened central region and a compression line on either side of the raised region. The raised thickened region increases the surface area of the napkin at the narrow waist region, whilst the compression lines inhibit the leakage of body fluid to the side edges of the napkin. In said document, the raised central region is between 10 and 40 mm thick. This may be considered as being too bulky by some potential wearers.

In WO 90/06096, side-edge leakage is addressed by the provision of a sanitary napkin having an absorption body, the density of which increases continuously from the edge margins in towards the centre part thereof. The configuration of such a napkin does not however lend itself to mass production in continuous webs.

An absorption body for use in a diaper or sanitary napkin is disclosed in SE-B-433 430 and consists of at least three superimposed layers. Each of the layers is compressed to varying degrees, with the inner layer exhibiting a plurality of longitudinally extending highty compressed zones.

EP-A-0 193 309 describes a paper diaper comprising, from the top, a fluff pulp surface layer, a holed or grooved partition layer, a diffusion crust layer and a fluff pulp main layer, all wrapped up in nonwoven fabric.

A sanitary napkin liner is known from US-A-4 795 455 which is designed to be used with a conventional sanitary napkin with a view to provide more rapid fluid take up and reduced fluid strike back. To hinder undesired lateral transmission of body fluids, the liner is provided with an hour-glass shaped embossing. In addition, and to promote rapid fluid absorbency, the napkin liner is provided with an embossed central channel of some 6 mm width. This channel directs rapid deposits of fluid along the channel, the fluid then being quickly transmitted through the liner to the underlying conventional sanitary napkin. Whilst the thus disclosed napkin liner is relatively thin, it is intended to be used in conjunction with a conventional napkin. In addition, to function optimally, it must be centrally positioned with respect to the wearer.

Similarly, US-A-4 624 666 describes a sanitary napkin having a central elongated channel for transporting body fluid generally in the elongated direction. Again, this napldn must be accurately positioned in order to function most effectively.

US-A-3 881 490 discloses later bonded absorbent pads having a thickness of from 1.6 mm to 6.3 mm and being embossed with a pattern of spaced-apart.

A method is disclosed in US-A-4 840 692 for producing an absorption body notably meant for use in cases of urinary incontinence in women. In said document it is an object to avoid using compressions in the body since these are said to slow down the rate of absorption of fluid. Instead, channels are formed in a pad of absorbent material by a working of cutting or milling. The pad is suitably 2 to 7 mm thick and the channels are said to need a width of at least 4 mm in order to facilitate distribution and absorption of the discharged fluid.

### SUMMARY OF THE INVENTION:

It is therefore an object of the invention to provide an absorption body, primarily though not exclusively for use in sanitary napkins, which body lends itself to economic mass production techniques, resists side leakage and is thinner than conventional napkins.

This object is achieved in accordance with the present invention by an absorption body having the features of Claim 1. Such an absorption body can be manufactured using the method according to claim 8.

The combination of a plurality of longitudinally extending channels and the specified absorbent material allows for an optimal distribution of discharged body fluid across a considerable area of the absorption body. Since the fluid is more evenly distributed, the absorption body can be made relatively thin and the risk of uncomfortable rewetting is accordingly reduced.

Advantageous embodiments of the absorption body and its method of manufacture are detailed in the respective dependent claims.

### BRIEF DESCRIPTION OF THE DRAWINGS:

The invention will now be described in greater detail by way of example only and with reference to the attached drawings, in which
- Fig. 1: is a schematic perspective view of an absorption body according to the invention;
- Fig. 2: is a schematic perspective view of a sanitary napkin, the topsheet of which is partially cut away to reveal the absorption body according to the invention, and
- Fig. 3: shows a peripheral region of an embossing roller used in the production of the absorption body according to the invention.

### DETAILED DESCRIPTION OF A PREFERRED EMBODIMENT:

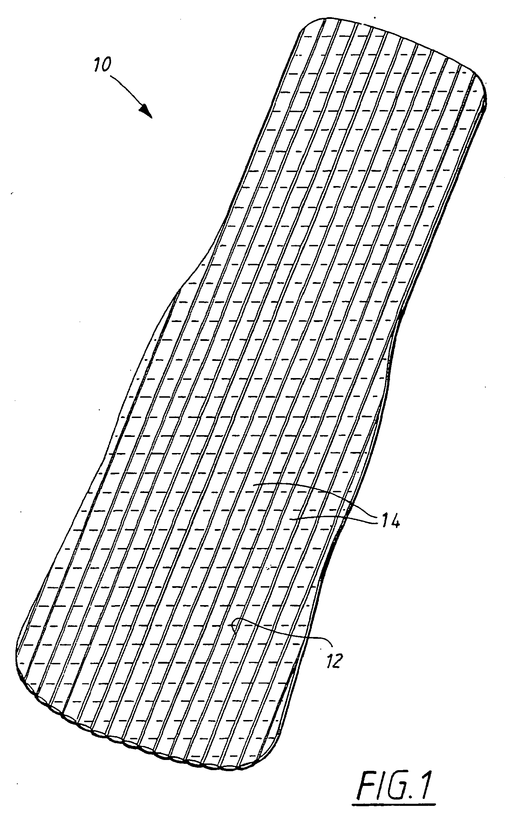

Referring to Fig. 1, reference numeral 10 generally denotes an absorption body incorporating the principles of the present invention. A preferred material for the absorption body 10 is airlaid latex-bonded paper. The principles of the invention may, however, be successfully realized with an absorption body employing a low density, thermally bonded airlaid paper comprising a mixture of absorbent fibres and non-wettable polymeric conjugate fibres.

The absorption body 10 may be made from a single layer of absorbent material or may be laminated. In accordance with the invention, the layer of absorbent material provides the absorption body with a basis weight of between about 80 and 240 g/m², preferably between about 120 and 160 g/m². Preferably, though, the absorption body is a laminate of two or more layers of absorbent material with lamination taking place between a pair of heated rollers. A particular advantage of having a laminated absorption body is that the individual layers of absorbent material which make up the body can be thinner than if the body were made from a single layer. This implies that greater lengths of material can be wound onto feed rolls which supply the absorbent material to the heated rollers, thereby reducing the frequency with which the feed rolls have to be changed.

In accordance with the present invention, the absorption body 10 is provided with at least three longitudinally extending embossed channels 12. Between adjacent channels, there are thereby formed longitudinally extending absorbent rib portions 14. Preferably, and as shown in Fig. 1, the channels 12 are more than three in number and are uniformally spaced across substantially the entire width of the absorption body. This allows the absorption body 10 to be made as a continuous web, with individual absorption bodies then being cut out therefrom. The primary purpose of the channels 12 is to aid the distribution of discharged body fluid across a considerable surface area of the absorption body 10. Tests have shown that good results are obtained if each of the embossed channels has a base width of between about 0.7 and 2.0 mm, preferably between about 0.8 and 1.2 mm, most preferably about 1 mm. The channels 12 are separated by a distance of between about 2.5 and 3.5 mm.

Since the absorption body of the present invention Is capable of distributing fluid across a considerable surface area, there is a reduced risk of a particular region of the body from becoming saturated earlier than other regions. It is therefore possible to make the absorption body thinner than conventional absorption bodies of the same material, whilst still maintaining substantially the same absorption capacity. When used in a sanitary napkin, for example, the absorption body 10 may have a thickness of between 0.5 and 1.2 mm, preferably between 0.8 and 1.1 mm.

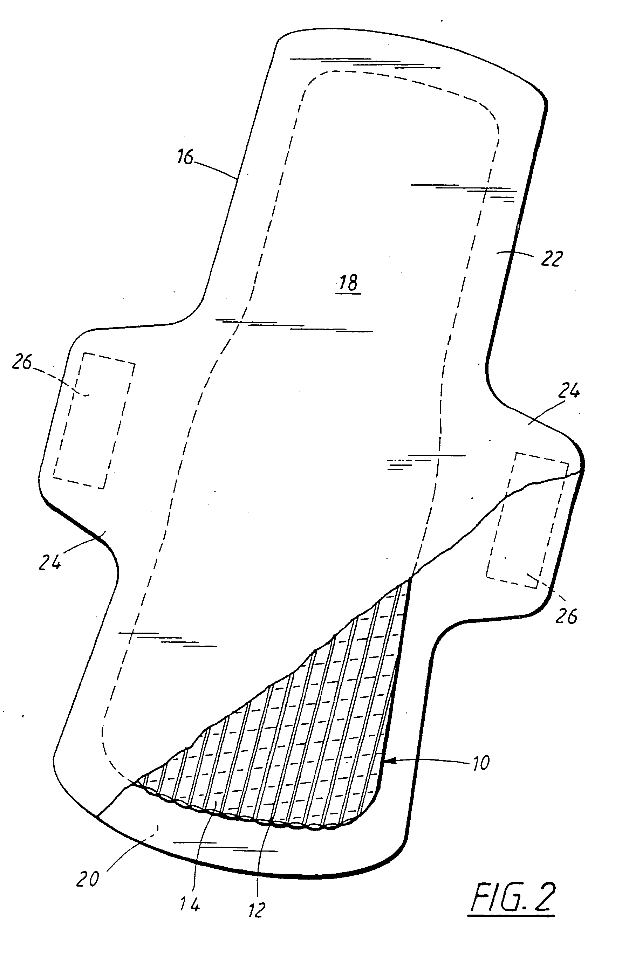

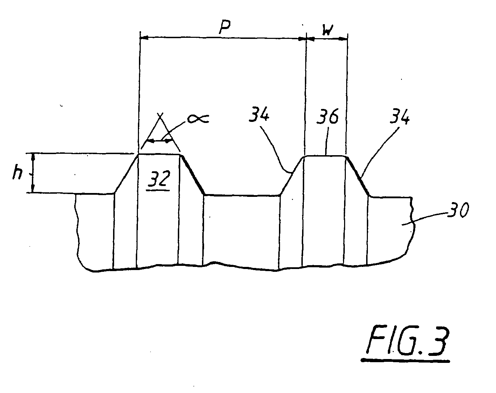

In Fig. 2 there is Illustrated a sanitary napkin 16 which Incorporates the absorption body 10 of the present invention. The sanitary napkin 16 comprises a topsheet 18 and a liquid-impermeable bottom sheet20, with the absorption body 10 sandwiched therebetween. The topsheet 18 is intended to face the body of the wearer when the napkin is worn and may be made from a multi-apertured plastics film of the type disclosed in US-A-4 626 254. As is evident from Fig. 2, the topsheet 18 and the bottom sheet 20 completely enclose the absorption body 10 to leave a peripheral margin 22 which is not occupied by the absorption body 10. The topsheet and the bottom sheet are laminated, preferably using an adhesive or, alternatively, using ultrasonic or thermobonding, along the peripheral margin 22 to thereby securely locate the absorption body within the napkin.

As is known in the art, the sanitary napkin 16 may be provided with one or more (not shown) strips of adhesive on the outer surface of the bottom sheet 20 to aid in the location of the napkin in the panties of the wearer. The strips of adhesive may of course be covered with a protective release strip which is provided to protect the adhesive from contamination and unintended adhesion prior to use. Similarly, the napkin 16 may have a pair of laterally positioned wings 24 with adhesive strips 26 on the portion of the bottom sheet 20 forming a part of said wings.

In Fig. 3, a peripheral region 30 of an embossing roller is shown which is used in the production of the absorption body according to the invention. The embossing roller is provided with a plurality of raised portions 32, each raised portion 32 comprising a pair of lateral side surfaces 34 converging towards a substantially flat peak 36. The number, size and spacing of the raised portions 32 determines the number, size and spacing of the channels 12 in the absorption body 10 when absorbent material is passed between the embossing roller and a smooth anvil roller.

Typically, each substantially flat peak 36 may have a width w of between about 0.7 mm and 2.0 mm, preferably between about 0.8 and 1.2 mm, and most preferably about 1 mm. The height h of the raised portions 32 is dependent on the desired thickness of the finished absorption body, though is typically about 1 mm: The lateral side surfaces 34 advantageously converge towards the peak 36 at an included angle α of between 45° and 75°, preferably about 60°. The spacing, or pitch p, between the raised portions 32 may typically lie between about 3 and 5 mm, preferably about 4 mm.

In order to aid the formation of the embossed channels 12, it is advantageous if the embossing process be conducted using an embossing roller surface temperature of about 120°C to 150°C, an anvil roller surface temperature.of about 50°C to 90°C, and an embossing pressure of between about 300 and 500 N/linear mm, preferably about 400 N/linear mm. These temperatures and pressures are sufficient to ensure satisfactory lamination of the absorption body when a plurality of layers of absorbent material are fed simultaneously through the pair of rollers.

The feed rate of the absorbent material through the rollers is primarily dependent on the thermal absorption properties of the absorbent material, though the number of layers, the temperature of the rollers and the embossing pressure also need to be considered. A suitable feed rate can typically lie between about 15 and 250 m/minute.

The present invention is not restricted to that described above and shown in the drawings, but may be varied within the scope of the appended claims. For example, the channels 12 need not be straight, i.e. they may be given a wave-shaped longitudinal form. In order to improve the absorption properties of the body, it is feasible that super-absorbent granulates or fibres be included in the absorption body. In such a case, the absorption body suitably has a basis weight of between about 110 and 170 g/m².

## Claims

1. An absorption body (10) suitable for use in sanitary napkins (16) or similar, said body comprising at least one layer of an absorbent material and presenting at least three longitudinally extending embossed channels (12), wherein said body (1 0) has a thickness of between 0.5 and 1.2 mm, an d in that said at least one layer of absorbent material is latex- or thermally-bonded airlaid paper which provides the absorption body (10) with a basis weight of between about 80 and 240 g/m² and wherein each of said embossed channels (12) has a base width of between about 0.7 and 2.0 mm. and wherein said channels (12) are uniformally spaced across substantially the entire width of the absorption body (10) and are separated by a distance of between about 2.5 and 3.5 mm.

2. The absorption body according to claim 1, **characterized in that** the absorption body (10) has a basis weight of between about 120 and 160 g/m².

3. The absorption body according to claim 1 or 2, **characterized in that** said body (10) is a laminate of two or more layers of absorbent material.

4. The absorption body according to any one of the preceding claims, **characterized in that** it has a thickness of between 0.8 and 1.1 mm.

5. The absorption body according to any one of the preceding claims, **characterized in that** said embossed channels (12) has a base width of between about 0.8 and 12 mm.

6. The absorption body according to claim 5, **characterized in that** said embossed channels (12) has a base width of about 1 mm.

7. The absorption body according to any, one of the preceding claims, **characterized in that** the material of the absorption body includes a super-absorbent substance and **in that** the material provides the absorption body (10) with a basis weight of between about 110 and 170 g/m².

8. A method of manufacturing an absorption body (10) as defined in any one of the preceding claims and suitable for use in sanitary napkins (16) or similar, said body comprising at least one layer of an absorbent material and presenting a plurality of longitudinally extending embossed channels (12), said method **characterized in that** said at least one layer of an absorbent material is latex- or thermally-bonded airlaid paperwhich provides the absorption body (10) with a basis weight of between about 80 and 240 g/m², **in that** said body (10).has a thickness of between 0.5 and 1.2 mm and **in that** said at least one layer of an absorbent material is passed between an embossing roller and an anvil roller, with said embossing roller being provided with at least three raised portions (32) to thereby create a corresponding number of channels (12) in the absorption body (10).

9. The method according to claim 8, **characterized in that** the surface of said embossing roller is maintained at a temperature of between about 120°C and 150°C, and **in that** the surface of said anvil roller is maintained at a temperature of between about 50°C and 90°C.

10. The method according to claim 8 or 9, **characterized in that** said raised portions (32) on the embossing roller each present a substantially flat peak (36) having a width (w) of between about 0.7 mm and 2.0 mm and **in that** said raised portions have a height (h) of about 1 mm.

11. The method according to claim 10, **characterized in that** the width (w) is between about 0.8 and 1.2 mm.

12. The method according to claim 11, **characterized in that** the width (w) is about 1 mm.

13. The method according to any one of claims 8 to 12. **characterized in that** said at least one layer of an absorbent material is fed between the anvil roller and the embossing roller at a rate of about 15 to 250 m/minute.

14. The method according to any one of claims 8 to 17, **characterized in that** an embossing pressure of between about 300 and 500 N/linear mm.

15. The method according to claim 14 **characterized in that** an embossing pressure of about 400 N/linear mm, is used.

16. The method according to any one of claims 8 to 15, **characterized in that** said embossing roller is provided with a plurality of substantially uniformly spaced raised portions (32) across substantially its entire width.

17. A sanitary napkin incorporating the absorption body according to any one of claims 1 to 7.

18. A sanitary napkin incorporating the absorption body manufactured by the method according to any one of claims 8 to 16.

## Patentansprüche

1. Absorbierender Körper (10), der zur Verwendung in Damenbinden (16) oder dergleichen geeignet ist, wobei der Körper wenigstens eine Schicht aus einem absorbierenden Material umfasst und wenigstens drei sich längs erstreckende geprägte Kanäle (12) aufweist, wobei der Körper (10) eine Dicke zwischen 0,5 und 1,2 mm hat und dass die wenigstens eine absorbierende Schicht aus absorbierendem Material Latex gebundenes oder thermisch gebundenes, airlaid Papier ist, das dem absorbierenden Körper (10) ein Flächengewicht zwischen ungefähr 80 und 240 g/m² verleiht und wobei jeder der geprägten Kanäle (12) eine Grundbreite zwischen 0,7 und 2,0 mm hat und wobei die Kanäle (12) über im Wesentlichen die gesamte Breite des absorbierenden Körpers (10) gleichmäßig beabstandet und durch einen Abstand von ungefähr 2,5 bis 3,5 mm voneinander getrennt sind.

2. Absorbierender Körper nach Anspruch 1, **dadurch gekennzeichnet, dass** der absorbierende Körper (10) ein Flächengewicht zwischen ungefähr 120 und 160 g/m² hat.

3. Absorbierender Körper nach Anspruch 1 oder 2, **dadurch gekennzeichnet, dass** der absorbierende Körper (10) ein Laminat aus zwei oder mehr Schichten aus absorbierendem Material ist.

4. Absorbierender Körper nach einem der vorhergehenden Ansprüche, **dadurch gekennzeichnet, dass** er eine Dicke zwischen 0,8 und 1,1 mm hat.

5. Absorbierender Körper nach einem der vorhergehenden Ansprüche, **dadurch gekennzeichnet, dass** jeder der geprägten Kanäle (12) eine Grundbreite zwischen 0,8 und 1,2 mm hat.

6. Absorbierender Körper nach Anspruch 5, **dadurch gekennzeichnet, dass** die geprägten Kanäle (12) eine Grundbreite von ungefähr 1 mm haben.

7. Absorbierender Körper nach einem der vorhergehenden Ansprüche, **dadurch gekennzeichnet, dass** das Material des absorbierenden Körpers eine superabsorbierende Substanz enthält und dass das Material dem absorbierenden Körper ein Flächengewicht von ungefähr 110 bis 170 g/m² verleiht.

8. Verfahren zur Herstellung eines absorbierenden Körpers (10), wie er in einem der voranstehenden Ansprüche definiert ist und der zur Verwendung in Damenbinden (16) oder dergleichen geeignet ist, wobei der Körper wenigstens eine Schicht aus einem absorbierenden Material umfasst und mehrere sich längs erstreckende geprägte Kanäle (12) aufweist, wobei das Verfahren **dadurch gekennzeichnet ist, dass** die wenigstens eine Schicht aus absorbierendem Material Latex gebundenes oder thermisch gebundenes, airlaid Papier ist, das dem absorbierenden Körper (10) ein Flächengewicht zwischen ungefähr 80 und 240 g/m² verleiht, dass der Körper (10) eine Dicke zwischen 0,5 und 1,2 mm hat und dass die wenigstens eine Schicht aus absorbierendem Material zwischen einer Prägewalze und einer Ambosswalze durchgeführt wird, wobei die Prägewalze mit wenigstens drei erhabenen Abschnitten (32) versehen ist, um in dem absorbierenden Körper (10) eine entsprechende Anzahl an Kanälen (12) zu erzeugen.

9. Verfahren nach Anspruch 8, **dadurch gekennzeichnet, dass** die Oberfläche der Prägewalze auf einer Temperatur von ungefähr 120 °C bis 150 °C gehalten wird und dass die Oberfläche der Ambosswalze auf einer Temperatur von ungefähr 50 °C bis 90 °C gehalten wird.

10. Verfahren nach Anspruch 8 oder 9, **dadurch gekennzeichnet, dass** jeder der erhabenen Abschnitte (32) auf der Prägewalze eine im Wesentlichen flache Spitze mit einer Breite (W) zwischen ungefähr 0,7 mm und 2,0 mm hat und dass die erhabenen Abschnitte eine Höhe (h) von ungefähr 1 mm haben.

11. Verfahren nach Anspruch 10, **dadurch gekennzeichnet, dass** die Breite (W) 0,8 mm bis 1,2 mm beträgt.

12. Verfahren nach Anspruch 11, **dadurch gekennzeichnet, dass** die Breite (W) ungefähr 1 mm beträgt.

13. Verfahren nach einem der Ansprüche 18 bis 12, **dadurch gekennzeichnet, dass** die wenigstens eine Schicht aus einem absorbierendem Material mit einer Geschwindigkeit von ungefähr 15 bis 250 m/min zwischen die Amboss- und Prägewalze eingeführt wird.

14. Verfahren nach einem der Ansprüche 8 bis 13, **dadurch gekennzeichnet, dass** ein Prägedruck von ungefähr 300 bis 500 N/linearem mm verwendet wird.

15. Verfahren nach Anspruch 14, **dadurch gekennzeichnet, dass** ein Prägedruck von ungefähr 400 N/linearem mm verwendet wird.

16. Verfahren nach einem der Ansprüche 8 bis 15, **dadurch gekennzeichnet, dass** die Prägewalze über im Wesentlichen ihre gesamte Breite mit einer Vielzahl von im Wesentlichen gleichmäßig beabstandeten, erhabenen Abschnitten (32) versehen ist.

17. Damenbinde, in der ein absorbierender Körper nach einem der Ansprüche 1-7 enthalten ist.

18. Damenbinde, in der ein nach einem der Ansprüche 8-16 hergestellter absorbierender Körper enthalten ist.

## Revendications

1. Corps d'absorption (10) adapté à une utilisation dans des serviettes hygiéniques (16) ou articles similaires, ledit corps comprenant au moins une couche de matériau absorbant et présentant au moins trois canaux estampés (12) s'étendant longitudinalement, dans lequel ledit corps (10) a une épaisseur comprise dans l'intervalle allant de 0,5 à 1,2 mm environ, et en ce que ladite couche de matériau absorbant au nombre d'au moins une est du papier déposé par air, collé au latex ou thermiquement, qui donne au corps d'absorption (10) un grammage compris dans l'intervalle allant de 80 à 240 g/m² environ, et dans lequel chacun desdits canaux estampés (12) a une largeur de base comprise dans l'intervalle allant de 0,7 à 2,0 mm environ, et dans lequel lesdits canaux (12) sont uniformément espacés sur sensiblement toute la largeur du corps d'absorption (10) et sont séparés d'une distance comprise dans l'intervalle allant de 2,5 à 3,5 mm environ.

2. Corps d'absorption selon la revendication 1, **caractérisé en ce que** le corps d'absorption (10) a un grammage compris dans l'intervalle allant de 120 à 160 g/m² environ.

3. Corps d'absorption selon la revendication 1 ou 2, **caractérisé en ce que** ledit corps (10) est une structure feuilletée de deux couches ou plus de matériau absorbant.

4. Corps d'absorption selon l'une quelconque des revendications précédentes, **caractérisé en ce que** son épaisseur est comprise dans l'intervalle allant de 0,8 à 1,1 mm.

5. Corps d'absorption selon l'une quelconque des revendications précédentes, **caractérisé en ce que** lesdits canaux estampés (12) ont une largeur de base comprise dans l'intervalle allant de 0,8 à 1,2 mm environ.

6. Corps d'absorption selon la revendication 5, **caractérisé en ce que** lesdits canaux estampés (12) ont une largeur de base d'environ 1 mm.

7. Corps d'absorption selon l'une quelconque des revendications précédentes, **caractérisé en ce que** le matériau du corps d'absorption comprend une substance super-absorbante et **en ce que** le matériau donne au corps d'absorption (10) un grammage compris dans l'intervalle allant de 110 à 170 g/m² environ.

8. Procédé de fabrication d'un corps d'absorption (10) tel que défini dans l'une quelconque des revendications précédentes et adapté à une utilisation dans des serviettes hygiéniques (16) ou articles similaires, ledit corps comprenant au moins une couche de matériau absorbant et présentant une pluralité de canaux estampés (12) s'étendant longitudinalement, ledit procédé étant **caractérisé en ce que** ladite couche de matériau absorbant au nombre d'au moins une est du papier déposé par air, collé au latex ou thermiquement, qui donne au corps d'absorption (10) un grammage compris dans l'intervalle allant de 80 à 240 g/m² environ, **en ce que** ledit corps (10) a une épaisseur comprise dans l'intervalle allant de 0,5 à 1,2 mm et **en ce que** l'on fait passer ladite couche de matériau absorbant au nombre d'au moins une entre un rouleau à estamper et un rouleau-enclume, ledit rouleau à estamper étant muni d'au moins trois parties saillantes (32) pour créer de ce fait un nombre correspondant de canaux (12) dans le corps d'absorption (10).

9. Procédé selon la revendication 8, **caractérisé en ce que** la surface dudit rouleau à estamper est maintenue à une température comprise dans l'intervalle allant d'environ 120 °C à 150 °C, et **en ce que** la surface dudit rouleau-enclume est maintenue à une température comprise dans l'intervalle allant d'environ 50 °C à 90 °C.

10. Procédé selon la revendication 8 ou 9, **caractérisé en ce que** lesdites parties saillantes (32) du rouleau à estamper présentent chacune un sommet sensiblement plat (36) dont la largeur (w) est comprise dans l'intervalle allant de 0,7 mm à 2,0 mm environ, et **en ce que** lesdites parties saillantes ont une hauteur (h) d'environ 1 mm.

11. Procédé selon la revendication 10, **caractérisé en ce que** la largeur (w) est comprise dans l'intervalle allant de 0,8 à 1,2 mm environ.

12. Procédé selon la revendication 11, **caractérisé en ce que** la largeur (w) est d'environ 1 mm.

13. Procédé selon l'une quelconque des revendications 8 à 12, **caractérisé en ce que** l'on fait avancer ladite couche de matériau absorbant au nombre d'au moins une entre le rouleau-enclume et le rouleau à estamper à un débit d'environ 15 à 250 m/minute.

14. Procédé selon l'une quelconque des revendications 8 à 13, **caractérisé en ce que** l'on utilise une pression d'estampage comprise dans l'intervalle allant de 300 à 500 N/mm linéaire environ.

15. Procédé selon la revendication 14, **caractérisé en ce que** l'on utilise une pression d'estampage de 400 N/mm linéaire environ.

16. Procédé selon l'une quelconque des revendications 8 à 15, **caractérisé en ce que** ledit rouleau à estamper est muni d'une pluralité de parties saillantes (32) espacées de manière sensiblement uniforme sur sensiblement toute sa largeur.

17. Serviette hygiénique comprenant le corps d'absorption selon l'une quelconque des revendications 1 à 7.

18. Serviette hygiénique comprenant le corps d'absorption fabriqué selon le procédé de l'une quelconque des revendications 8 à 16.
